# EUROPEAN PATENT SPECIFICATION

(11) **EP 4 308 599 B1**
(45) Date of publication and mention of the grant of the patent: **18.11.2026**
(21) Application number: 22714729.5
(22) Date of filing: 17.03.2022
(51) Int. Cl.: C07K 16/00, A61P 35/00, A61K 31/00, G01N 33/50

(54) **PDGFR-ALPHA INHIBITORS FOR USE IN TREATING LEIOMYOSARCOMAS WITH A PDGFR-BETA NEGATIVE STATUS**
PDGFR-ALPHA INHIBITOREN ZUR VERWENDUNG BEI DER BEHANDLUNG VON LEIOMYOSARKOMEN MIT EINEM PDGFR-BETA NEGATIVEN STATUS
INHIBITEURS DE PDGFR-ALPHA POUR UTILISATION DANS LE TRAITEMENT DE LÉIOMYOSARCOMES PRÉSENTANT UN STATUT PDGFR-BETA NÉGATIF

(30) Priority: 19.03.2021 US 202163163426 P
(43) Date of publication of application: 24.01.2024
(60) Divisional application: 26210554.7
(73) Proprietor: Eli Lilly and Company, Indianapolis, IN 46285 (US)
(72) Inventor: OAKLEY, Gerard Joseph III, Indianapolis, Indiana 46206-6288 (US); PETERSON, Patrick Marshall, Indianapolis, Indiana 46206-6288 (US); STANCATO, Louis Frank, Indianapolis, Indiana 46206-6288 (US); WALGREN, Richard Anthony, Indianapolis, Indiana 46206-6288 (US)
(74) Representative: Eli Lilly and Company Limited
(86) International application number: PCT/US2022/020761
(87) International publication number: WO 2022/197929

(56) References cited:
- WO-A1-2016/003789
- WO-A1-2018/169779
- WO-A1-2019/231803
- WO-A2-2007/021860
- US-A- 5 620 687
- CHIOREAN E GABRIELA ET AL: "A phase I study of olaratumab, an anti-platelet-derived growth factor receptor alpha (PDGFR[alpha]) monoclonal antibody, in patients with advanced solid tu", CANCER CHEMOTHERAPY AND PHARMACOLOGY, SPRINGER VERLAG , BERLIN, DE, vol. 73, no. 3, 23 January 2014 (2014-01-23), pages 595 - 604, XP035339661, ISSN: 0344-5704, [retrieved on 20140123], DOI: 10.1007/S00280-014-2389-9
- WEHLER THOMAS C ET AL: "PDGFRa/beta expression correlates with the metastatic behavior of human colorectal cancer: A possible rationale for a molecular targeting strategy", ONCOLOGY REPORTS,, vol. 19, no. 3, 29 February 2008 (2008-02-29), pages 697 - 704, XP009500026, ISSN: 1021-335X, [retrieved on 20080301], DOI: 10.3892/OR.19.3.697

## Description

The present disclosure relates to the field of cancer. More specifically, the present disclosure relates to the treatment of cancer patients with platelet derived growth factor receptor alpha ("PDGFRα") inhibiting compounds. Even more particularly, the present disclosure relates to the treatment of cancer patients with PDGFRα inhibiting compounds, wherein the cancer patients are identified as being PDGFR beta ("PDGFRβ") negative.

Cancer is a disease with extensive histoclinical heterogeneity including wide variations in tumor morphology and physiology. Although some conventional histologic and clinical features have been correlated to prognosis, the vast heterogeneity across the forms of cancer, spanning from the cellular to the tissue level, impacts response to therapy and subsequent benefit to the patient. Therefore, selectively treating cancer patients who will benefit from a particular treatment continues to pose a challenge.

PDGFRα inhibiting compounds have shown promise as a therapeutic for cancer in preclinical and clinical studies. Despite this promise, PDGFRα inhibiting compounds have failed to meet therapeutic endpoints in some oncology clinical trials. For example, in clinical trials for treating soft tissue sarcoma, LARTRUVO^{®}, an antibody that specifically binds human PDGFRα, failed to meet certain therapeutic endpoints. Thus, a need exists for improved methods for treating patients with PDGFRα inhibiting compounds. In particular, such methods should provide prognostic, diagnostic or predictive value for cancer patients treated with PDGFRα inhibiting compounds. The present disclosure addresses this need by providing methods of treating cancer patients with PDGFRα inhibiting compounds.

WO 2016/003789 discloses the treatment of soft tissue sarcomas such as leiomyosarcoma with Lartruvo in combination with doxorubicin.

The present invention provides an effective amount of a PDGFRα inhibiting compound for use in the treatment of cancer in a patient, wherein the patient is identified as having a cancer that is human PDGFRβ negative,
wherein the PDGFRα inhibiting compound is an antibody, wherein the antibody specifically binds PDGFRα;
wherein the antibody which specifically binds PDGFRα comprises a heavy chain (HC) and a light chain (LC), wherein the HC comprises SEQ ID NO: 1 and the LC comprises SEQ ID NO: 2;
wherein the antibody which specifically binds PDGFRα is administered in simultaneous, separate, or sequential combination with doxorubicin;
wherein the cancer is soft tissue sarcoma;
wherein the soft tissue sarcoma is leiomyosarcoma; and
wherein the patient is female and wherein the female is determined to have a PDGFRβ negative cancer. The invention is defined by the appended claims.

Disclosed herein is also an *in vitro* method to predict if a female leiomyosarcoma patient will have a positive outcome to treatment with olaratumab in combination with doxorubicin, comprising the steps:
determining the PDGFR beta status in a tumour sample of the patient comprising:
   a. contacting a biological sample from the cancer patient with an antibody or antigen-binding fragment thereof that specifically binds human PDGFRβ, wherein a complex of the PDGFRβ antibody or antigen-binding fragment thereof and human PDGFRβ is formed;
   b. removing any non-specifically bound first antibody or antigen-binding fragment thereof;
   c. detecting and quantifying the human PDGFRβ in the biological sample;
wherein the antibody or antigen binding fragments thereof which specifically binds PDGFRα comprises a heavy chain (HC) and a light chain (LC),
wherein the HC comprises SEQ ID NO: 1 and the LC comprises SEQ ID NO: 2; and wherein if the patient is determined to be negative for the expression of PDGFR beta, the patient is predicted to have a positive outcome of the treatment.

The present disclosure, surprisingly provides methods of treating cancer patients with PDGFRα inhibiting compounds which provide prognostic, diagnostic or predictive value for cancer patients treated with PDGFRα inhibiting compounds. More particularly, embodiments of the present disclosure provide methods for treating cancer patients having a cancer that is human PDGFRβ negative by administering a PDGFRα inhibiting compound.

Embodiments of the present disclosure further provide a method of treating cancer in a patient in need thereof, comprising administering to the patient an effective amount of a PDGFRα inhibiting compound. Specifically, embodiments of the present disclosure provide methods of treating cancer in a patient in need thereof, comprising administering to the patient an effective amount of a PDGFRα inhibiting compound, wherein the patient is identified as having a cancer that is human PDGFRβ negative. In yet another embodiment, the present disclosure provides a method of treating a patient having a human PDGFRβ negative cancer, comprising administering to the patient an effective amount of a PDGFRα inhibiting compound.

Accordingly, embodiments of the present disclosure provide methods of treating cancer in a patient comprising identifying a patient as having a cancer that is human PDGFRβ negative. In an embodiment the present disclosure provides a method of treating cancer in a patient in need thereof, comprising identifying the patient as having a cancer that is human PDGFRβ negative, and administering an effective amount of a PDGFRα inhibiting compound to the patient. In a further embodiment the present disclosure provides a method of treating cancer in a patient in need thereof, by administering to the patient an effective amount of a PDGFRα inhibiting compound, wherein the patient is identified as having a cancer that is human PDGFRβ negative and human PDGFRα positive.

In some embodiments of the present disclosure methods of identifying a patient as having a cancer that is human PDGFRβ negative comprise, contacting a biological sample from the patient with an antibody that specifically binds human PDGFRβ, and detecting binding of the antibody to human PDGFRβ in the biological sample.

According to embodiments of the present disclosure, a method of detecting PDGFRβ in a biological sample is provided. Such methods comprise performing an assay on a biological sample from the patient. Embodiments of the present disclosure further provide a method comprising contacting the biological sample with an antibody that specifically binds human PDGFRβ and detecting binding of the antibody to human PDGFRβ in the biological sample.

According to embodiments of the present disclosure, a method of diagnosing a patient with cancer as in need of treatment with a PDGFRα inhibiting compound is provided. Such methods comprise identifying the patient as having a cancer that is human PDGFRβ negative. Such methods further comprise performing an assay on a biological sample from the patient. Embodiments of the present disclosure further provide a method comprising contacting the biological sample with an antibody that specifically binds human PDGFRβ and detecting binding of the antibody to human PDGFRβ in the biological sample.

According to embodiments of the present disclosure, a method of quantifying human PDGFRβ in a biological sample is provided. Such methods comprise contacting a biological sample from a patient with an antibody that specifically binds human PDGFRβ and detecting binding of the antibody to human PDGFRβ in the biological sample.

In an embodiment of the present disclosure, the biological sample is determined to be PDGFRβ negative when PDGFRβ in the biological sample is determined to be present in less than about 10% of tumor cells of the biological sample. In yet other embodiments the biological sample is determined to be PDGFRβ positive when PDGFRβ in the biological sample is determined to be present in greater than or equal to about 10% of tumor cells of the biological sample. In a further embodiment of the present disclosure the patient is administered a PDGFRα inhibiting compound if the biological sample from the patient is determined to be PDGFRβ negative.

In particular embodiments the present disclosure provides a method of diagnosing a cancer patient as in need of treatment with a PDGFRα inhibiting compound, comprising the steps of: obtaining a biological sample from the patient; contacting the biological sample with an antibody or antigen-binding fragment thereof that specifically binds human PDGFRβ, wherein a complex of the antibody or antigen-binding fragment thereof and human PDGFRβ is formed; contacting with a second antibody or antigen binding fragment thereof, the complex of the human PDGFRβ antibody or antigen-binding fragment thereof and human PDGFRβ, wherein the second antibody comprises a detectable label; detecting a signal provided by said detectable label; and wherein, if the biological sample from the cancer patient is determined as PDGFRβ negative the cancer patient is diagnosed as in need of treatment with a PDGFRα inhibiting compound. In a further embodiment the present disclosure comprises the step of administering to the cancer patient an effective amount of a PDGFRα inhibiting compound, if the biological sample is determined to be PDGFRβ negative.

An embodiment of the present disclosure, provides an in vitro method of diagnosing a cancer patient as in need of treatment with an antibody or antigen binding fragments thereof, that specifically binds human PDGFRα, comprising: obtaining a biological sample from the patient; contacting the biological sample with an antibody or antigen-binding fragment thereof that specifically binds human PDGFRβ, wherein a complex of the PDGFRβ antibody or antigen-binding fragment thereof and human PDGFRβ is formed; removing any non-specifically bound PDGFRβ antibody or antigen-binding fragment thereof; detecting and quantifying the human PDGFRβ in the biological sample; and wherein, if the biological sample from the cancer patient is determined to be PDGFRβ negative the cancer patient is diagnosed as in need of treatment with an antibody or antigen binding fragment thereof, that specifically binds human PDGFRα. In yet further embodiments the step of detecting human PDGFRβ in the biological sample comprises detecting with a second antibody or antigen binding fragment thereof, the complex of the PDGFRβ antibody or antigen-binding fragment thereof and human PDGFRβ in the biological sample. In yet even further embodiments of the present disclosure, at least one of the PDGFRβ antibody or antigen binding fragment thereof, or the second antibody or antigen binding fragment thereof, comprises a detectable label. In yet a further embodiment said step of detecting human PDGFRβ in the biological sample comprises detecting a signal provided by the detectable label upon formation of the complex comprising, the PDGFRβ antibody and human PDGFRβ or the second antibody and human PDGFRβ. In yet a further embodiment said step of detecting human PDGFRβ in the biological sample comprises detecting a signal provided by the detectable label upon formation of the complex comprising, the antibody, human PDGFRβ, and the second antibody.

Further embodiments of the present disclosure comprise the step of administering to the cancer patient an effective amount of an antibody specifically binding PDGFRα, if the biological sample is determined to be PDGFRβ negative.

In embodiments of the present disclosure the PDGFRα inhibiting compound is an antibody or an antigen binding fragment thereof. In other embodiments of the present disclosure the PDGFRα inhibiting compound is a small molecule inhibitor. In particular embodiments, the PDGFRα inhibiting compound is an antibody that specifically binds PDGFRα. In even more particular embodiments, the antibody specifically binding PDGFRα is olaratumab. In some embodiments the PDGFRα inhibiting compound is an antibody drug conjugate. In some embodiments the PDGFRα inhibiting compound is an antibody where the antibody is labeled with a radiopharmaceutical targeting agent.

According to some embodiments, antibodies are provided that specifically bind PDGFRα. In more specific embodiments, the antibody that specifically binds PDGFRα comprises a heavy chain variable region (VH) and a light chain variable region (VL), wherein the VH comprises heavy chain complementarity determining regions (HCDR) HCDR1, HCDR2, and HCDR3, and the VL comprises light chain complementarity determining regions (LCDR) LCDR1, LCDR2, and LCDR3, wherein: the HCDR1 comprises SEQ ID NO: 5, the HCDR2 comprises SEQ ID NO: 6, the HCDR3 comprises SEQ ID NO: 7, the LCDR1 comprises SEQ ID NO: 8, the LCDR2 comprises SEQ ID NO: 9, and the LCDR3 comprises SEQ ID NO: 10. In a further embodiment, the VH of the antibody that specifically binds PDGFRα comprises SEQ ID NO: 3 and the VL comprises SEQ ID NO: 4. In yet even a further embodiment, the antibody which specifically binds PDGFRα comprises a heavy chain (HC) and a light chain (LC), wherein the HC comprises SEQ ID NO: 1 and the LC comprises SEQ ID NO: 2. In yet a particular embodiment, the antibody which specifically binds PDGFRα is olaratumab.

In yet further embodiments of the present disclosure, an effective amount of antibody or antigen binding fragment thereof, that specifically binds PDGFRα is administered to a patient identified as having a cancer that is human PDGFRβ negative. In such embodiments an effective amount of antibody or antigen binding fragment thereof is administered to the patient identified as having a cancer that is human PDGFRβ negative at a loading dose of about 15 mg/kg, or about 20 mg/kg, or about 25 mg/kg on each of day 1 and day 8 of a first 21-day cycle or on each of day 1 and day 8 of a first 28-day cycle, followed by administering a standard dose of the antibody or antigen binding fragment thereof to the patient, at about 15 mg/kg, about 20 mg/kg, or about 25 mg/kg on each of day 1 and day 8 of a subsequent 21-day cycle or on each of day 1 and day 8 of a subsequent 28-day cycle. In yet a further embodiment the antibody or antigen binding fragment thereof is administered in simultaneous, separate, or sequential combination with one or more chemotherapeutic agents. In an embodiment the chemotherapeutic agent comprises at least one of nab-paclitaxel, doxorubicin, gemcitabine, or docetaxel.

In yet further embodiments of the present disclosure, an effective amount of olaratumab is administered to a patient identified as having a cancer that is human PDGFRβ negative. In such embodiments an effective amount of olaratumab is administered to the patient identified as having a cancer that is human PDGFRβ negative, at a loading dose of about 15 mg/kg, or about 20 mg/kg, or about 25 mg/kg, on each of day 1 and day 8 of a first 21-day cycle or on each of day 1 and day 8 of a first 28-day cycle, followed by administering a standard dose of olaratumab to the patient at about 15 mg/kg, about 20 mg/kg, or about 25 mg/kg, on each of day 1 and day 8 of a subsequent 21-day cycle or on each of day 1 and day 8 of a subsequent 28-day cycle. In yet a further embodiment olaratumab is administered in simultaneous, separate, or sequential combination with one or more chemotherapeutic agents. In an embodiment the chemotherapeutic agent comprises at least one of nab-paclitaxel, doxorubicin, gemcitabine, or docetaxel.

In some embodiments of the present disclosure, the cancer determined as PDGFRβ negative is soft tissue sarcoma, pancreatic cancer, endometrial cancer, ovarian cancer, osteosarcoma, chondrosarcoma, rhabdomyosarcoma, breast cancer, bone cancer, or prostate cancer. In some embodiments the cancer is leiomyosarcoma. In some embodiments the cancer is liposarcoma. In an embodiment of the present disclosure the cancer is a primary tumor. In an embodiment the cancer is a metastatic cancer. In yet other embodiments the cancer has metastasized. In particular embodiments of the present disclosure the patient is female, and the female is determined to have a PDGFRβ negative cancer.

Platelet derived growth factor receptor alpha (PDGFRα) and platelet derived growth factor receptor beta (PDGFRβ) belong to the type III tyrosine kinase receptor (RTK) family and are implicated in various cancer types. PDGFRα has been considered as a relevant factor in tumor proliferation, angiogenesis, and metastatic dissemination in various cancer types.

The terms "PDGFR alpha inhibiting compound" or "PDGFR alpha inhibitor" as used interchangeably herein, is a compound that decreases, blocks, inhibits, abrogates, or interferes with signal transduction resulting from the interaction of PDGFRα with either one or more of its ligands or binding partners. PDGFRα inhibiting compounds can be extracellular inhibitors or intracellular inhibitors and more than one inhibitor may be employed. Extracellular inhibitors include, but are not limited to, compounds that bind to PDGFRα or one or more of its ligands (for example, PDGF-AA, -AB, -BB, -CC). Intracellular inhibitors include, but are not limited to, small molecule receptor tyrosine kinase inhibitors. Non-limiting examples of PDGFRα inhibiting compounds include antibodies, antigen binding fragments thereof, small molecule inhibitors, antibody drug conjugates, fusion proteins, immunoadhesin molecules, and oligopeptides.

The terms "antibody," and 'antigen binding fragments thereof' as used herein, refers to an immunoglobulin molecule that specifically binds an antigen. In an embodiment the antibody or antigen binding fragment thereof specifically binds PDGFRα. An exemplary antibody of the present disclosure is an immunoglobulin G type 1 (IgG1) antibody or antigen binding fragment thereof. According to particular embodiments, such antibody or antigen binding fragment thereof, comprises a heavy chain variable region (VH) and a light chain variable region (VL), wherein the VH comprises CDRs HCDR1, HCDR2 and HCDR3 and the VL comprises complementarity determining regions (CDRs) LCDR1, LCDR2, and LCDR3 wherein HCDR1 has the amino acid sequence of SEQ ID NO: 3, HCDR2 has the amino acid sequence of SEQ ID NO: 4, and HCDR3 has the amino acid sequence of SEQ ID NO: 5, LCDR1 has the amino acid sequence of SEQ ID NO: 6, LCDR2 has the amino acid sequence of SEQ ID NO: 7, and LCDR3 has the amino acid sequence of SEQ ID NO: 8. According to some embodiments the antibody or antigen binding fragment thereof provided by the present disclosure, the VH has the amino acid sequence of SEQ ID NO: 3 and the VL has the amino acid sequence of SEQ ID NO: 4. According to some embodiments the antibody or antigen binding fragment thereof provided by the present disclosure, comprises a light chain (LC) and a heavy chain (HC) wherein the HC has the amino acid sequence of SEQ ID NO: 1 and the LC has the amino acid sequence of SEQ ID NO: 2. In an embodiment of the present disclosure the antibody is olaratumab.

According to some embodiments, antibodies of the present disclosure may be humanized. In some embodiments, antibodies of the present disclosure comprise an IgG1 heavy chain. In some embodiments, antibodies of the present disclosure comprise a kappa light chain. According to even further embodiments, the present disclosure provides pharmaceutical compositions comprising an antibody of the present disclosure and one or more pharmaceutically acceptable carriers, diluents or excipients.

Embodiments of an antibody include a monoclonal antibody, polyclonal antibody, human antibody, humanized antibody, chimeric antibody, bispecific or multispecific antibody, or conjugated antibody. The antibodies can be of any class (e.g., IgG, IgE, IgM, IgD, IgA), and any subclass (e.g., IgG1, IgG2, IgG3, IgG4).

Assignment of amino acid residues to the CDRs may be done according to the well-known schemes, including those described in Kabat (Kabat et al., "Sequences of Proteins of Immunological Interest," National Institutes of Health, Bethesda, Md. (1991)), Chothia (Chothia et al., "Canonical structures for the hypervariable regions of immunoglobulins", Journal of Molecular Biology, 196, 901-917 (1987); Al-Lazikani et al., "Standard conformations for the canonical structures of immunoglobulins", Journal of Molecular Biology, 273, 927-948 (1997)), North (North et al., "A New Clustering of Antibody CDR Loop Conformations", Journal of Molecular Biology, 406, 228-256 (2011)), or IMGT (the international ImMunoGeneTics database available on at www.imgt.org; see Lefranc et al., Nucleic Acids Res. 1999; 27:209-212).

The term "specifically binds PDGFRα" or "binds PDGFRα" as used herein, refers to an interaction of an antibody with an epitope region of human PDGFRα as provided in e.g., NCBI reference sequence P16234.1 (SEQ ID NO: 11). As used herein, the term "specifically binds PDGFRβ" or "binds PDGFRβ" refers to an interaction of an antibody with an epitope region of human PDGFRβ as provided in e.g., NCBI reference sequence P09619.1 (SEQ ID NO: 12).

The terms "PDGFRβ negative" or "PDGFRβ positive" as used herein, refer to whether the form of cancer of a patient is a PDGFRβ negative or PDGFRβ positive form of cancer. As detailed herein, whether the form of cancer of a patient is a PDGFRβ negative or PDGFRβ positive form of cancer may be determined based on a qualitative or quantitative determination. According to embodiments here, whether the form of cancer of a patient is a PDGFRβ negative or PDGFRβ positive form of cancer may be determined based on an assessment of approximate levels of PDGFRβ present in a biological sample from a cancer patient when compared to a reference value. According to a more particular embodiment, a patient is determined to have a PDGFRβ negative form of cancer when the approximate level of PDGFRβ present in the biological sample is less than about 10% of tumor cells in a biological sample from the patient, as determined by an IHC assay. In another embodiment, a patient is determined to have a PDGFRβ negative form of cancer based on levels of PDGFRβ as determined by a grading system using reference value(s).

Levels of PDGFRβ, as provided by assays of the present disclosure or assays known in the art, may be absolute values (e.g., level within a biological sample) or relative values (e.g., level compared to a reference). Levels of PDGFRβ in tumor cells can be evaluated via assays including, but not limited to, immunohistochemistry (IHC), polymerase chain reaction (PCR), quantitative, qualitative or semi-quantitative reverse transcription PCR (RT-PCR), applications of automated or semi-automated image analysis of IHC or other quantitative/semi-quantitative/qualitative assessments of protein expression or mRNA expression, artificial intelligence analysis of scanned slides or other laboratory acquired data for protein expression, brightfield in situ hybridization (BRISH), fluorescent in situ hybridization (RNA FISH), protein immunofluorescence, quantitative/semi-quantitative/qualitative proteomics methods, cytological assays, and RNA sequencing.

A "reference value" as used herein refers to a known, or approximate level of a reference value that can be an absolute or relative level, a range, a minimum level, a mean level, a threshold level, and/or a median level. Additionally, a reference value can also serve as a baseline or threshold value. According to a particular embodiment as used herein, a "reference value" of PDGFRβ indicates whether a form of cancer of a patient is a PDGFRβ negative or positive form of cancer.

The terms "biological sample" or "patient sample" used interchangeably herein, refers to a human sample. Non-limiting sources of a biological sample for use in the present invention include cancer, tumors, tumor biopsy. biopsy aspirates, solid tissues, tumor cells, and metastatic, migrating, circulating tumor cells. Additionally, biological sample may also refer to blood, plasma, serum, lymph fluid, ascites, fluidic extracts, the external sections of the skin, respiratory, nasal, intestinal, and genitourinary tracts, tears, saliva, milk, organs, cell cultures and / or cell culture constituents.

The term "about" as used herein, means within 5%.

The term "cancer" as used herein, is a disease pathologically characterized by the physiological condition in a mammal that is typically characterized by unregulated cell proliferation, immortality, metastatic potential, rapid growth and proliferation rate, and/or certain characteristic morphological features. Often, cancer cells are in the form of a tumor, but such cells may exist alone or may circulate in the blood stream as independent cells, such as leukemic cells, or metastatic, migrating, or circulating, tumor cells. The cancer may be a solid tumor or a leukemia. Tumors may be benign, malignant, or dormant and may also be characterized as primary tumors or metastatic tumors. In some embodiments, non-limiting examples of cancer include soft tissue sarcoma, pancreatic cancer, endometrial cancer, osteosarcoma, chondrosarcoma, rhabdomyosarcoma, breast cancer, bone cancer, prostate cancer, gastrointestinal cancer, colon cancer, squamous cell carcinoma, head and neck cancer, small-cell lung cancer, non-small cell lung cancer, glioblastoma, cervical cancer, ovarian cancer, bladder cancer, hepatoma, colorectal cancer, salivary gland carcinoma, kidney cancer, vulval cancer, thyroid cancer, hepatic cancer, and/or laryngeal cancer.

The terms "soft tissue sarcoma" or "STS" as used herein, is a type of cancer that begins in the tissues that connect, support and surround other body structures. This includes fat, muscle, fibrous tissues, blood vessels, nerves, tendons, linings of joints, or deep skin tissues, and/or the lining of joints. They can be found in any part of the body. More than 50 subtypes of soft tissue sarcoma exist. Types of STS include, but are not limited to, angiosarcoma, dermatofibrosarcoma protuberans, epithelioid sarcoma, gastrointestinal stromal tumor (GIST), Kaposi's sarcoma, liposarcoma, malignant peripheral nerve sheath tumors, myxofibrosarcoma, rhabdomyosarcoma, solitary fibrous tumor, synovial sarcoma, or undifferentiated pleomorphic sarcoma.

Chemotherapeutic agents are chemical agents or drugs that are selectively destructive to cancer cells and tissues. Chemotherapeutics may include but are not limited to compounds such as, taxane compounds, compounds that act via taxane mechanisms, platinum compounds, anthracycline compounds, antimetabolites, epipodophyllotoxin compounds, camptothecin compounds, or any combination thereof. Chemotherapy drugs can be administered alone or in combination with other therapeutic agents. In some embodiments a chemotherapeutic agent comprises nab-paclitaxel, doxorubicin, docetaxel, or gemcitabine.

The term "diagnosis" as used herein, is used to refer to the identification or classification of a molecular or pathological state, disease or condition (e.g., cancer). For example, "diagnosis" may refer to identification of a particular type of cancer. "Diagnosis" may also refer to the classification of a particular subtype of cancer, e.g., by histopathological criteria, or by molecular features (e.g., a subtype characterized by expression of one or a combination of biomarkers (e.g., particular genes or proteins encoded by said genes, or expression levels of particular genes or proteins encoded by said genes)).

Embodiments of the present disclosure also pertain to methods of clinical diagnosis, or prognosis, of a subject performed by a medical professional using the methods disclosed herein. The methods, as described herein, can, for example, be performed by an individual, a health professional, or a third party, for example a service provider who interprets information from the subject. As explained herein, a medical professional may initiate or modify treatment after receiving information regarding a diagnostic method of the present disclosure. For example, a medical professional may recommend a therapy, a change in therapy or an additional diagnostic assessment.

The terms "treat" or "treating" or "treatment" as used herein, refer to processes involving a slowing, interrupting, arresting, controlling, stopping, reducing, regressing, and/or reversing the progression or severity of an existing disease such as cancer, but does not necessarily involve a total elimination of the disease, or disease state.

The term an "effective amount" as used herein, refers to an amount of a protein or nucleic acid or vector or composition or inhibiting compound that will elicit the biological or medical response of a subject, for example, reduction or inhibition of an enzyme or a protein activity, or ameliorate symptoms, alleviate conditions, slow or delay disease progression, or prevent a disease, etc. In a non-limiting embodiment, the term "effective amount" refers to an amount necessary (at dosages and for periods of time and for the means of administration) of a protein or nucleic acid or vector or composition or inhibiting compound that, when administered to a subject, is effective to at least partially alleviate, inhibit, prevent and/or ameliorate a condition, or a disorder or a disease to achieve the desired therapeutic result. An effective amount of the protein or nucleic acid or vector or composition or inhibiting compound may vary according to factors such as the disease type and state, age, sex, and weight of the individual, and the ability of the protein or nucleic acid or vector or composition, or therapeutic, such as an antibody, to elicit a desired response in the individual. An effective amount is also one in which any toxic or detrimental effects of the protein or nucleic acid or vector or composition or inhibiting compound of the present invention are outweighed by the therapeutically beneficial effects.

The terms "patient," "subject," and "individual," as used interchangeably herein, refers to a human. In certain embodiments, the patient is further characterized with a disease, disorder, or condition (e.g., cancer). In another embodiment, the patient is further characterized as being at risk of developing a disorder, disease, or condition (metastasis, growth, spread of the cancer or tumor) and would benefit from a reduction in the risk of metastasis, growth, spread of the cancer or tumor.

An antibody for use of the present invention can be incorporated into a pharmaceutical composition which can be prepared by methods well known in the art and comprise an antibody of the present invention and one or more pharmaceutically acceptable carrier(s) and/or diluent(s) (e.g., Remington, The Science and Practice of Pharmacy, 22nd Edition, Loyd V., Ed., Pharmaceutical Press, 2012, which provides a compendium of formulation techniques as are generally known to practitioners). Suitable carriers for pharmaceutical compositions include any material which, when combined with an antibody for use of the present invention, retains the molecule's activity and is non-reactive with the patient's immune system. A pharmaceutical composition comprising an antibody for use of the present invention can be administered to a patient at risk for, or exhibiting, diseases or disorders as described herein by parental routes (e.g., intravenous, subcutaneous, intraperitoneal, intramuscular, or transdermal).

### EXAMPLES

### Example 1: Assessment of Overall Survival in PDGFRβ negative advanced or metastatic Soft Tissue Sarcoma patients

***Study Design:*** Patients with advanced or metastatic soft tissue sarcoma are treated with olaratumab (20 mg/kg loading dose on Days 1 and 8 of a 21-day cycle in Cycle 1, followed by 15 mg/kg on Days 1 and 8 in subsequent 21-day cycle) in combination with doxorubicin (75 mg/m² on Day 1) ("investigational cohort") and compared to patients treated with placebo (on Days 1 and 8) plus doxorubicin (75 mg/m² on Day 1) ("control cohort"). Patients are treated for 8 cycles, followed by olaratumab monotherapy or placebo until evidence of progressive disease, unacceptable toxicity or death. PDGFRβ tumor expression status of patients is determined substantially as described herein.

Patients are assessed for median overall survival (OS).

***Methods of determining PDGFRβ expression:*** PDGFRβ expression in tumor cells can be evaluated via methods including, but not limited to, immunohistochemistry, quantitative, qualitative or semi-quantitative reverse transcription PCR (RT-PCR), applications of automated or semi-automated image analysis of IHC or other quantitative/semi-quantitative/qualitative assessments of protein expression, artificial intelligence analysis of scanned slides or other laboratory acquired data for protein expression, brightfield in situ hybridization (BRISH), fluorescent in situ hybridization (RNA FISH), protein immunofluorescence, quantitative/semi-quantitative/qualitative proteomics methods and RNA sequencing.

***Immunohistochemistry Assay to determine PDGFRβ expression:*** For immunohistochemistry analysis, tumor tissue from patients is collected, formalin-fixed in 10% neutral buffered formalin, and paraffin-embedded (FFPE). PDGFRβ protein expression on tumor cells is assessed by immunohistochemistry. Briefly, from FFPE tissue blocks containing the patient tumor tissue, a 4-6 micrometer section is obtained and placed on a positively charged glass slide. Anti-PDGFRβ mouse monoclonal antibody 2B3 is used to detect expression of PDGFRβ (clone 2B3, Cell Signaling Technology^{®} catalog number 3175S), diluted in Dako Primary Antibody Diluent with Background Reducing Components (Dako/Agilent catalog number S3022) at 0.25 µg/mL. Immunohistochemistry is performed on a Dako Autostainer Link 48/PT Link Incubator. Deparaffinization with the Link 48/PT Link Incubator is accomplished at 97 °C for 20 minutes. Target retrieval is next accomplished with immersion of the unstained slides into EnVision^{™} FLEX Target Retrieval Solution High pH (Dako) on the Dako Link48/PT Link Incubator. Following a rinse in RT EnVision^{™} FLEX Wash Buffer (1x), specific immunohistochemical staining with the 2B3 antibody is accomplished by FLEX Peroxidase Block for 5 minutes, application of the 0.25 ug/mL concentration of anti-human PDGFRβ antibody 2B3 with incubation for 60 minutes, followed by FLEX/HRP application and incubation for 20 minutes, then application of FLEX DAB+ Substrate Chromogen for 10 minutes, and finally FLEX hematoxylin for 5 minutes. Ready-to-use FLEX Mouse Negative Control (Dako/Agilent catalog number IR750) is performed alongside PDGFRβ staining and used as a quality control (negative control) for the assay. Stained slides are then evaluated by trained personnel using a brightfield microscope. PDGFRβ tumor expression status is provided dichotomously as "positive" or "negative", where a "positive" result is defined as samples where at least 10% of the tumor cells present (rounded to the nearest decile) demonstrate at least weak but specific membranous staining (1+ on a 0, 1+, 2+, 3+ scale of staining intensity, with 1+ being weakest but still specific membrane staining and 3+ being strong and diffuse membrane staining). "Negative" corresponded to staining that did not meet these criteria.

### Results:

***STS Patients:*** As demonstrated in Table 1, STS patients identified as having PDGFRβ negative tumor status had a significant improvement in median OS of 28.32 months in the investigational cohort when compared to 20.57 months for patients in the control cohort (HR=0.85 [95% CI: 0.54-1.33] p=0.4861). Furthermore, patients in the investigational cohort identified as having both PDGFRβ negative tumors status and PDGFRα positive tumor status also showed a significant improvement in median OS of 28.5 months (N=66) vs. 20.6 months (N=75) in the control cohort. However, no significant differences in median OS between the investigational and control cohort were observed in patients whose tumor status was identified as, PDGFRβ positive (18.8 months vs 19.9 months respectively for the investigational and control cohort), PDGFRα positive (17.2 months vs 19.1 months respectively for the investigational and control cohort), and PDGFRα negative (23.6 months vs 21.9 months respectively for the investigational and control cohort).

**Table 1: Median OS in STS Patients**

| **Category of Patients** | **Olaratumab + Doxorubicin** | | **Placebo + Doxorubicin** | | |
|---|---|---|---|---|---|
| | **N** | **Median OS months (95% CI)** | **N** | **Median OS months (95% CI)** | **HR (95% CI) p-value** |
| **PDGFRβ negative** | 66 | 28.32 (14.42 - NA) | 75 | 20.57 (16.49 - NA) | 0.85 (0.54 - 1.33) p=0.4861 |
| **PDGFRβ positive** | 163 | 18.79 (15.24 - 21.19) | 160 | 19.88 (15.57 - 23.75) | 1.10 (0.84 - 1.44) p=0.4759 |

| | | | | | |
|---|---|---|---|---|---|
| N = patients treated in investigation cohort or control cohort; OS = Overall Survival; HR = Hazard Ratio; CI = confidence interval, p-value = Stratified Log-rank p-value. | | | | | |

***LMS Patients:*** As demonstrated in Table 2, LMS patients in the investigational cohort identified as having PFGDRβ negative tumor status had a significant improvement in median OS of 29.11 months (N=29) compared to 21.88 months (N=37) in the control cohort (HR=0.65 [95% CI: 0.33-1.25; p=0.1970). However, no differences in median OS in LMS patients identified as having PDGFRβ positive tumor status was observed between the investigational cohort (20.14 months; N=77) and the control cohort (21.39 months; N=73) (HR=1.05 [95% CI: 0.71-1.55; p=0.7951).

***LMS Female Patients:*** Analysis of PDGFRβ status in LMS female patients, showed a significant improvement in OS HR in the subpopulation of LMS female patients identified as having PDGFRβ negative tumor status (0.55; N = 53; p = 0.14) when compared to OS HR for LMS female patients identified as having PDGFRβ positive tumor status (1.34; N = 115; p = 0.20). Further adjusting for ECOG PS, the OS HR for PDGFRβ positive women with LMS was 1.34 (N = 115; p = 0.20), while the OS HR for PDGFRβ negative women with LMS was 0.55 (N = 53; p= 0.14) . This difference in OS HR between PDGFRβ positive and negative women with LMS resulted in a statistically significant "treatment by PDGFRβ " interaction (N = 168; p = 0.040).

***LMS Patients, where LMS Female Patients having PDGFRβ positive tumor status are excluded:*** As demonstrated in Table 2, LMS patients, where the LMS Female Patients identified as having PDGFRβ positive tumor status are excluded from the LMS Median OS analysis, a significant overall survival benefit in the investigational cohort of 28.5 months (N=58) is observed when compared to 20.9 months (N=61) in the control cohort (HR = 0.60; N=119; p = 0.035). This interaction of PDGFRβ expression status is not observed for men with LMS.

**Table 2. Median OS in LMS Patients**

| **Category of Patients** | **Olaratumab + Doxorubicin** | **Placebo + Doxorubicin** | | | |
|---|---|---|---|---|---|
| | **N** | **Median OS months (95% CI)** | **N** | **Median OS months (95% CI)** | **Hazard Ratio (95% CI) p-value** |
| **LMS PDGFRβ negative** | 29 | 29.11 (20.73, NA) | 37 | 21.88 (16.72, 35.55) | 0.65 (0.33, 1.25) p=0.1970 |
| **LMS PDGFRβ positive** | 77 | 20.14 (13.08, 22.11) | 73 | 21.39 (15.57, 24.57) | 1.05 ((0.71, 1.55) p=0.7951 |
| **LMS Patients excluding PDGFRβ positive Females** | 58 | 28.5 | 61 | 20.9 | 0.60; p = 0.035 |

| | | | | | |
|---|---|---|---|---|---|
| N = patients treated in investigation cohort or control cohort; OS = Overall Survival; HR = Hazard Ratio; CI = confidence interval; p-value = stratified Log-rank p-value. | | | | | |

### Example 2: Assessment of Overall Survival in PDGFRβ negative non-resectable metastatic Pancreatic Cancer patients

***Study Design:*** Median overall survival of patients with PDGFRβ negative non-resectable metastatic Pancreatic cancer are treated with olaratumab in a dose escalation schedule (15 mg/kg, 20 mg/kg, or 25 mg/kg administered on days 1, 8, and 15 of a 28-day cycle) in combination with nab-paclitaxel and gemcitabine (administered on days 1, 8, and 15 for a 28-day cycle per USPI insert). Patients with non-resectable metastatic Pancreatic cancer are treated with olaratumab on days 1, 8, and 15 of a 28-day cycle followed by administration of nab-paclitaxel (125 mg/m2), and gemcitabine (1000 mg/m2) on days 1, 8, and 15 of each 28-day cycle. Patients are assessed for overall survival.

### Example 3: Assessment of Overall Survival in PDGFRβ negative advanced Soft Tissue Sarcoma patients

***Study Design:*** Median overall survival of patients with PDGFRβ negative advanced or metastatic soft tissue sarcoma are treated with olaratumab in a dose escalation study at 15 mg/kg of olaratumab (administered on days 1 and 8) or 20 mg/kg of olaratumab (administered on days 1 and 8), in combination with gemcitabine administered at 900 mg/m2 on days 1 and 8 and docetaxel administered at 75 mg/m2 on day 8 of a 21-day cycle. Patients are assessed for overall survival.

### SEQUENCES

**SEQ ID NO: 1 (HC of human PDGFR alpha antibody)** 
MGWSCIILFLVATATGVHSQLQLQESGPGLVKPSETLSLTCTVSGGSINSSSYYWG
WLRQSPGKGLEWIGSFFYTGSTYYNPSLRSRLTISVDTSKNQFSLMLSSVTAADT
AVYYCARQSTYYYGSGNYYGWFDRWDQGTLVTVSSASTKGPSVFPLAPSSKSTS
GGTAALGCLVKDYFPEPVTVSWNSGALTSGVHTFPAVLQSSGLYSLSSVVTVPSS
SLGTQTYICNVNHKPSNTKVDKRVEPKSCDKTHTCPPCPAPELLGGPSVFLFPPKP
KDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKL
TVDKSRWQQGNVFSCSVMHEALHNHYTQKSLSLSPGK
**SEQ ID NO: 2 (LC of human PDGFR alpha antibody)** 
MGWSCIILFLVATATGVHSEIVLTQSPATLSLSPGERATLSCRASQSVSSYLAWYQ
QKPGQAPRLLIYDASNRATGIPARFSGSGSGTDFTLTISSLEPEDFAVYYCQQRSN
WPPAFGQGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLS
SPVTKSFNRGEC
**SEQ ID NO: 3 (VH of human PDGFR alpha antibody)** 
QLQLQESGPGLVKPSETLSLTCTVSGGSINSSSYYWGWLRQSPGKGLEWIGSFFY
TGSTYYNPSLRSRLTISVDTSKNQFSLMLSSVTAADTAVYYCARQSTYYYGSGNY
YGWFDRWDQGTLVTVSS
**SEQ ID NO: 4 (VL of human PDGFR alpha antibody)** 
EIVLTQSPATLSLSPGERATLSCRASQSVSSYLAWYQQKPGQAPRLLIYDASNRAT
GIPARFSGSGSGTDFTLTISSLEPEDFAVYYCQQRSNWPPAFGQGTKVEIK
**SEQ ID NO: 5 (HCDR1 of human PDGFR alpha antibody)**
   SSSYY
**SEQ ID NO: 6 (HCDR2 of human PDGFR alpha antibody)**
   SFFYTGSTYYNPSLRS
**SEQ ID NO: 7 (HCDR3 of human PDGFR alpha antibody)**
   QSTYYYGSGNYYGWFDR
**SEQ ID NO: 8 (LCDR1 of human PDGFR alpha antibody)**
   RASQSVSSYLA
**SEQ ID NO: 9 (LCDR2 of anti-human PDGFR alpha antibody)**
   DASNRAT
**SEQ ID NO: 10 (LCDR3 of human PDGFR alpha antibody)**
   QQRSNWPPA
**SEQ ID NO: 11 (human PDGFR alpha)**
**SEQ ID NO: 12 (human PDGFR beta)**

## Claims

1. An effective amount of a PDGFRα inhibiting compound for use in the treatment of cancer in a patient, wherein the patient is identified as having a cancer that is human PDGFRβ negative,
wherein the PDGFRα inhibiting compound is an antibody, wherein the antibody specifically binds PDGFRα;
wherein the antibody which specifically binds PDGFRα comprises a heavy chain (HC) and a light chain (LC), wherein the HC comprises SEQ ID NO: 1 and the LC comprises SEQ ID NO: 2;
wherein the antibody which specifically binds PDGFRα is administered in simultaneous, separate, or sequential combination with doxorubicin;
wherein the cancer is soft tissue sarcoma;
wherein the soft tissue sarcoma is leiomyosarcoma; and
wherein the patient is female and wherein the female is determined to have a PDGFRβ negative cancer.

2. An effective amount of a PDGFRα inhibiting compound for use according to claim 1, wherein the patient is identified as having a cancer that is human PDGFRβ negative by performing an assay on a biological sample from the patient.

3. An effective amount of a PDGFRα inhibiting compound for use according to claim 2, wherein the biological sample comprises tissue or bodily fluid.

4. An effective amount of a PDGFRα inhibiting compound for use according to claim 3, wherein the tissue comprises tumor tissue.

5. An effective amount of a PDGFRα inhibiting compound for use according to claim 3, wherein the bodily fluid comprises blood, plasma, or serum.

6. An effective amount of a PDGFRα inhibiting compound for use according to any one of claims 2 to 5, wherein the assay comprises performing an *in vitro* assay on the biological sample.

7. An effective amount of a PDGFRα inhibiting compound for use according to claim 6, wherein the *in vitro* assay comprises:
(a) a histological assay, or cytological assay; or
(b) an immunoassay or polymerase chain reaction assay.

8. An effective amount of a PDGFRα inhibiting compound for use according to any one of claims 2 to 7, wherein, the biological sample is contacted with an antibody, the antibody specifically binds human PDGFRβ, and binding of the antibody to human PDGFRβ is detected in the biological sample.

9. An effective amount of a PDGFRα inhibiting compound for use according to claim 8, wherein the human PDGFRβ is quantified in the biological sample and it is determined whether the biological sample is PDGFRβ negative.

10. An effective amount of a PDGFRα inhibiting compound for use according to claim 9, wherein the biological sample is determined to be PDGFRβ negative when PDGFRβ in the biological sample is determined to be present in less than 10% of tumor cells of the biological sample.

11. An effective amount of a PDGFRα inhibiting compound for use according to any one of claims 1 to 10, wherein the antibody which specifically binds PDGFRα is olaratumab.

12. An effective amount of a PDGFRα inhibiting compound for use according to claim 11, wherein an effective amount of olaratumab is administered to the patient, at a loading dose of about 15 mg/kg, or about 20 mg/kg, or about 25 mg/kg, on each of day 1 and day 8 of a first 21-day cycle or on each of day 1 and day 8 of a first 28-day cycle, followed by administering a standard dose of olaratumab at about 15 mg/kg, about 20 mg/kg, or about 25 mg/kg, on each of day 1 and day 8 of a subsequent 21-day cycle or on each of day 1 and day 8 of a subsequent 28-day cycle.

13. An effective amount of a PDGFRα inhibiting compound for use according to any one of claims 1 to 12, wherein the cancer is metastatic cancer.

## Patentansprüche

1. Wirksame Menge einer PDGFRα-hemmenden Verbindung zur Verwendung bei der Behandlung von Krebs bei einem Patienten, wobei der Patient als einen Krebs aufweisend identifiziert ist, der für humanes PDGFRβ negativ ist,
wobei die PDGFRα-inhibierende Verbindung ein Antikörper ist, wobei der Antikörper spezifisch an PDGFRα bindet;
wobei der Antikörper, der spezifisch an PDGFRα bindet, eine schwere Kette (HC) und eine leichte Kette (LC) umfasst, wobei die HC SEQ ID NO: 1 umfasst und die LC SEQ ID NO: 2 umfasst;
wobei der Antikörper, der spezifisch an PDGFRα bindet, in gleichzeitiger, getrennter oder sequenzieller Kombination mit Doxorubicin verabreicht wird;
wobei der Krebs ein Weichteilsarkom ist;
wobei das Weichteilsarkom Leiomyosarkom ist; und
wobei der Patient weiblich ist und wobei bestimmt wird, dass die weibliche Person einen PDGFRβ-negativen Krebs aufweist.

2. Wirksame Menge einer PDGFRα-inhibierenden Verbindung zur Verwendung nach Anspruch 1, wobei der Patient durch Durchführen eines Assays an einer biologischen Probe des Patienten als einen Krebs aufweisend identifiziert wird, der human-PDGFRβ-negativ ist.

3. Wirksame Menge einer PDGFRα-hemmenden Verbindung zur Verwendung nach Anspruch 2, wobei die biologische Probe Gewebe oder Körperfluid umfasst.

4. Wirksame Menge einer PDGFRα-hemmenden Verbindung zur Verwendung nach Anspruch 3, wobei das Gewebe Tumorgewebe umfasst.

5. Wirksame Menge einer PDGFRα-hemmenden Verbindung zur Verwendung nach Anspruch 3, wobei das Körperfluid Blut, Plasma oder Serum umfasst.

6. Wirksame Menge einer PDGFRα-hemmenden Verbindung zur Verwendung nach einem der Ansprüche 2 bis 5, wobei der Assay das Durchführen eines *in vitro*-Assays an der biologischen Probe umfasst.

7. Wirksame Menge einer PDGFRα-hemmenden Verbindung zur Verwendung nach Anspruch 6, wobei der *in vitro*-Assay Folgendes umfasst:
(a) ein histologisches Assay oder zytologisches Assay; oder
(b) ein Immunoassay oder Polymerasekettenreaktionsassay.

8. Wirksame Menge einer PDGFRα-inhibierenden Verbindung zur Verwendung nach einem der Ansprüche 2 bis 7, wobei die biologische Probe mit einem Antikörper in Kontakt gebracht wird, der Antikörper spezifisch an humanes PDGFRβ bindet und die Bindung des Antikörpers an humanes PDGFRβ in der biologischen Probe nachgewiesen wird.

9. Wirksame Menge einer PDGFRα-hemmenden Verbindung zur Verwendung nach Anspruch 8, wobei das humane PDGFRβ in der biologischen Probe quantifiziert wird und bestimmt wird, ob die biologische Probe PDGFRβ-negativ ist.

10. Wirksame Menge einer PDGFRα-hemmenden Verbindung zur Verwendung nach Anspruch 9, wobei die biologische Probe als PDGFRβ-negativ bestimmt wird, wenn bestimmt wird, dass PDGFRβ in der biologischen Probe in weniger als 10 % der Tumorzellen der biologischen Probe vorhanden ist.

11. Wirksame Menge einer PDGFRα-hemmenden Verbindung zur Verwendung nach einem der Ansprüche 1 bis 10, wobei der Antikörper, der spezifisch an PDGFRα bindet, Olaratumab ist.

12. Wirksame Menge einer PDGFRα-hemmenden Verbindung zur Verwendung nach Anspruch 11, wobei dem Patienten eine wirksame Menge an Olaratumab in einer Beladungsdosis von etwa 15 mg/kg oder etwa 20 mg/kg oder etwa 25 mg/kg jeweils an Tag 1 und Tag 8 eines ersten 21-Tage-Zyklus oder jeweils an Tag 1 und Tag 8 eines ersten 28-Tage-Zyklus verabreicht wird, gefolgt von der Verabreichung einer Standarddosis von Olaratumab von etwa 15 mg/kg, etwa 20 mg/kg oder etwa 25 mg/kg jeweils an Tag 1 und Tag 8 eines nachfolgenden 21-Tage-Zyklus oder jeweils an Tag 1 und Tag 8 eines nachfolgenden 28-Tage-Zyklus.

13. Wirksame Menge einer PDGFRα-hemmenden Verbindung zur Verwendung nach einem der Ansprüche 1 bis 12, wobei es sich bei dem Krebs um metastasierten Krebs handelt.

## Revendications

1. Quantité efficace d'un composé inhibiteur de PDGFRα pour utilisation dans le traitement du cancer chez un patient, dans laquelle le patient est identifié comme ayant un cancer qui est négatif pour le PDGFRβ humain,
dans laquelle le composé inhibiteur du PDGFRα est un anticorps, dans laquelle l'anticorps se lie spécifiquement au PDGFRα ;
dans laquelle l'anticorps qui se lie spécifiquement à PDGFRα comprend une chaîne lourde (HC) et une chaîne légère (LC), dans laquelle la HC comprend la SEQ ID NO : 1 et le LC comprend SEQ ID NO : 2 ;
dans laquelle l'anticorps qui se lie spécifiquement au PDGFRα est administré en combinaison simultanée, séparée ou séquentielle avec la doxorubicine ;
dans laquelle le cancer est un sarcome de tissus mous ;
dans laquelle le sarcome de tissus mous est un léiomyosarcome ; et
dans laquelle le patient est une femme et dans lequel il est déterminé que la femme est atteinte d'un cancer PDGFRβ négatif.

2. Quantité efficace d'un composé inhibiteur de PDGFRα pour utilisation selon la revendication 1, dans laquelle le patient est identifié comme étant atteint d'un cancer qui est négatif pour le PDGFRβ humain en effectuant un dosage sur un échantillon biologique du patient.

3. Quantité efficace d'un composé inhibiteur du PDGFRα pour utilisation selon la revendication 2, dans laquelle l'échantillon biologique comprend un tissu ou un fluide corporel.

4. Quantité efficace d'un composé inhibiteur de PDGFRα pour utilisation selon la revendication 3, dans laquelle le tissu comprend du tissu tumoral.

5. Quantité efficace d'un composé inhibiteur du PDGFRα pour utilisation selon la revendication 3, dans laquelle le fluide corporel comprend du sang, du plasma ou du sérum.

6. Quantité efficace d'un composé inhibiteur de PDGFRα pour utilisation selon l'une quelconque des revendications 2 à 5, dans laquelle l'essai comprend le fait d'effectuer un essai *in vitro* sur l'échantillon biologique.

7. Quantité efficace d'un composé inhibiteur de PDGFRα pour utilisation selon la revendication 6, dans laquelle l'essai *in vitro* comprend :
(a) une analyse histologique, ou une analyse cytologique ; ou
(b) un dosage immunologique ou un dosage par réaction en chaîne par polymérase.

8. Quantité efficace d'un composé inhibiteur du PDGFRα pour utilisation selon l'une quelconque des revendications 2 à 7, dans laquelle l'échantillon biologique est mis en contact avec un anticorps, l'anticorps se lie spécifiquement au PDGFRβ humain, et la liaison de l'anticorps au PDGFRβ humain est détectée dans l'échantillon biologique.

9. Quantité efficace d'un composé inhibiteur de PDGFRα pour utilisation selon la revendication 8, dans laquelle le PDGFRβ humain est quantifié dans l'échantillon biologique et il est déterminé si l'échantillon biologique est PDGFRβ négatif.

10. Quantité efficace d'un composé inhibiteur de PDGFRα pour utilisation selon la revendication 9, dans laquelle l'échantillon biologique est déterminé comme étant négatif pour PDGFRβ lorsque le PDGFRβ dans l'échantillon biologique est déterminé comme étant présent dans moins de 10 % des cellules tumorales de l'échantillon biologique.

11. Quantité efficace d'un composé inhibiteur du PDGFRα pour utilisation selon l'une quelconque des revendications 1 à 10, dans laquelle l'anticorps qui se lie spécifiquement au PDGFRα est l'olaratumab.

12. Quantité efficace d'un composé inhibiteur du PDGFRα pour utilisation selon la revendication 11, dans laquelle une quantité efficace d'olaratumab est administrée au patient, à une dose de charge d'environ 15 mg/kg, ou d'environ 20 mg/kg, ou d'environ 25 mg/kg, à chacun des jours 1 et 8 d'un premier cycle de 21 jours ou à chacun des jours 1 et 8 d'un premier cycle de 28 jours, suivie de l'administration d'une dose standard d'olaratumab à environ 15 mg/kg, environ 20 mg/kg, ou environ 25 mg/kg, à chacun des jours 1 et 8 d'un cycle ultérieur de 21 jours ou à chacun des jours 1 et 8 d'un cycle ultérieur de 28 jours.

13. Quantité efficace d'un composé inhibiteur de PDGFRα pour utilisation selon l'une quelconque des revendications 1 à 12, dans laquelle le cancer est un cancer métastatique.
